# EUROPEAN PATENT APPLICATION

(11) **EP 1 535 994 A1**
(43) Date of publication of application: **01.06.2005**
(21) Application number: 03792761.3
(22) Date of filing: 21.08.2003
(51) Int. Cl.: C12N 5/06

(54) **HUMAN BONE STEM CELLS**

(30) Priority: 23.08.2002 JP 2002244280
(71) Applicant: SRL, INC., Tachikawa-shi, Tokyo 190-8567 (JP); Sakuragawa, Norio, Kodaira-shi, Tokyo 187-0032 (JP); UCHIDA, Saiko, Bunkyo-ku, Tokyo 113-0022 (JP)
(72) Inventor: SAKURAGAWA, Norio, Kodaira-shi, Tokyo 187-0032 (JP); UCHIDA, Saiko, Bunkyo-ku, Tokyo 113-0022 (JP)
(74) Representative: Cripps, Joanna Elizabeth
(86) International application number: PCT/JP2003/010554
(87) International publication number: WO 2004/018658

(57) **Abstract**

A bone stem cell which may be supplied stably and which is free from the problem about the compatibility in transplantation is disclosed. The bone stem cell according to the present invention is separated from human amniotic mesenchymal cell layer. The bone stem cell may be used for osteogenesis in a bone defect or the like.

## Description

### Technical Field

The present invention relates to a novel bone stem cell which was separated from human amnion. The cell according to the present invention is useful for bone repair or the like, by transplantation of the cell.

### Background Art

Conventionally, in cases where bone repair is needed due to injury, removal of bone tumor or the like, autologous bone such as thigh bone is collected from the patient himself and transplanted. However, with this method, the burden of the patient is very heavy. On the other hand, in the fields of organ reconstruction and tissue engineering, bone repair by transplantation of stem cells (bone stem cells) which may differentiate into bone cells is now studied. Bone stem cells have been discovered in bone marrow and fat cell layer. However, stable supply thereof is problematic. Further, there is a problem that when transplanting these bone stem cells, to prevent rejection reaction, compatibility must be checked, and the cells cannot be transplanted to an incompatible patient.

### Disclosure of the Invention

An object of the present invention is to provide a bone stem cell which may be supplied stably and which is free from the problem of the compatibility in transplantation.

The present inventors intensively studied to discover that bone stem cells exist in human amniotic mesenchymal layer, thereby completing the present invention.

That is, the present invention provides a bone stem cell separated from human amniotic mesenchymal cell layer. The present invention also provides cells for forming bone cells, which comprise bone stem cells existing in human amniotic mesenchymal cell layer. The present invention further provides a method for obtaining bone cells comprising culturing (a) bone stem cell(s) existing in human amniotic mesenchymal cell layer in a bone cell-differentiation medium. The present invention still further provides a method for osteogenesis comprising transplanting bone stem cells existing in human amniotic mesenchymal cell layer into (a) bone defect(s). The present invention still further provides use of a bone stem cell existing in human amniotic mesenchymal cell layer for osteogenesis.

By the present invention, a bone stem cell existing in human amniotic mesenchymal layer was first provided. Since the bone stem cell according the present invention is originated from amnion, it can be stably supplied, and there is no problem about the compatibility in transplantation.

### Best Mode for Carrying out the Invention

As mentioned above, the cells according to the present invention are separated from human amniotic mesenchymal cell layer. The mesenchymal cell layer is located between the chorionic membrane layer and amniotic epithelial cell layer. Although amniotic membrane is a tissue originated from the fetus, it can be recovered in the state of being attached to placenta originated from mother. Further, it is a large tissue which covers the entire inner wall of uterus. Therefore, they can be obtained in a large amount. Further, since placenta and amnion attached thereto are discarded as medical wastes, there is no ethical problem in the collection of amnion.

The cells according to the present invention may be separated by peeling the amniotic epithelial cell layer + mesenchymal cell layer of human amnion from chorionic membrane layer, treating the resultant with trypsin to remove amniotic epithelial cells, and by treating the resultant with a protease. Preferred examples of the treatment with the protease include treatments with a mixture of papain, collagenase, neutral protease + DNase (see Example below), but not restricted thereto. In the cells separated by the treatment with the protease, cells other than bone stem cells are also included. On the other hand, the cell surface antigen recognized by a monoclonal antibody (SB-10) is expressed before the cell is differentiated into bone cell, and disappears after differentiation into bone cell (Bruder SP et al., J Bone Mineral Res 13: 655, 1998). Therefore, by using a flow cytometry system using SB-10, separation and culturing of bone stem cells may be attained. In the present invention, a cell expressing alkaline phosphatase is judged as a bone cell. This judgment is accepted in this field (Jaiswal N et al., J Cell Biochem 64: 295, 1997; Pittenger MF et al., Science 284: 143, 1999).

As the bone cell-differentiation medium used for the differentiation of the bone stem cells into bone cells, known bone cell-differentiation media may be employed. A preferred example of the bone cell-differentiation media is a medium (Pittenger MF et al., Science 284:143,1999) containing 100 nM dexamethasone, 10 mM β-glycerol phosphate, 0.25 mM ascorbate and 10% FBS (fetal bovine serum) in DMEM (Dulbecco's modified Eagle's medium). Although the culturing conditions are not restricted, it is preferred to culture the cells at 37°C which is the body temperature of human for 2 to 4 weeks. It is preferred to carry out the culturing in the atmosphere of 5% CO₂ gas.

Cultured cells obtained by primary culture or subculture of the above-mentioned cell according to the present invention, which can be differentiated into the cells expressing alkaline phosphatase, are also within the scope of the present invention.

The cells according to the present invention are originated from human amnion and the amnion is originated from the fetus, so that the cells are immunologically tolerant. That is, by immunohistostaining, the cells according to the present invention are HLA Class I positive and HLA Class II negative. Further, Fas ligand-positive cells exist. Recently, it is thought that the reason why the amniotic tissue hardly induces rejection is that HLA Class 1b (HLA-G) is expressed and Fas ligand-positive cell exist (Ophthalmology, 42:257-269, 2000). Thus, the cells according to the present invention may be transplanted without the problem of HLA compatibility.

The cells according to the present invention may be used for repair or reconstruction of bone by transplanting the cells as they are or after differentiation into bone cells expressing alkaline phosphatase. The site to which the cells are to be transplanted is not restricted, and usually, a bone defect caused by injury, removal of bone tumor or the like, for which the repair or reconstruction of the bone is desired. The transplantation may be carried out in the same manner as in the known transplantation of bone stem cells. The number of cells to be transplanted is appropriately selected depending on the size of the bone defect, symptom and so on, it is usually appropriate to transplant about 10³ to 10⁷ cells.

### Examples

The present invention will now be described by way of examples thereof. It should be noted, however, that the present invention is not restricted to the following Examples.

### Example 1

### 1. Separation and Culture of Cells

After obtaining informed consent of a postnatal mother, from human placenta, the amniotic epithelial cell layer + mesenchymal cell layer were obtained by separating the layers from the chorionic membrane layer. The separated layers were treated with 0.125% trypsin solution + 1.3 mM EDTA at 37°C for 15 minutes.
After repeating this treatment 4 times, the cells were collected by centrifuging the trypsin solution, and the cells were washed 3 times with phosphate buffer (PBS) (trypsin-treated fraction (Comparative Example 1)). The tissue block which was not digested by this treatment was washed with PBS and then treated under shaking with a mixed enzyme solution (0.01 wt% papain, 1 mg/ml collagenase, 0.01 wt% DNase, 0.1 wt% neutral protease) at 37°C for 1 hour. The resultant was centrifuged at 1000 rpm for 10 minutes and the obtained precipitate was suspended in PBS. After passing the suspension through a 20 µm filter, the cells were washed three times with PBS (mixed enzyme-treated fraction).

The obtained cells in the mixed enzyme-treated fraction were cultured in DMEM medium (Pittenger et al., Science 284:143,1999) containing 100 nM dexamethasone, 10 mM β-glycerol phosphate, 0.25 mM ascorbate and 10% FBS in a culture dish under 5% CO₂ atmosphere at 37°C. The medium was replaced after 3 to 4 days.

After culturing the cells for 21 days, production of alkaline phosphatase was histologically checked using a commercially available alkaline phosphatase detection kit (Sigma kit 85, produced by Sigma). The histological detection of alkaline phosphatase was carried out in accordance with the manufacturer's instruction of the commercially available kit.

As a result, alkaline phosphatase was clearly detected. By this, it was confirmed that the cells according to the present invention are bone stem cells which may be differentiated into bone cells.

## Claims

1. A bone stem cell separated from human amniotic mesenchymal cell layer.

2. Cells for forming bone cells, which comprise bone stem cells existing in human amniotic mesenchymal cell layer.

3. A method for obtaining bone cells comprising culturing (a) bone stem cell(s) existing in human amniotic mesenchymal cell layer in a bone cell-differentiation medium.

4. A method for osteogenesis comprising transplanting bone stem cells existing in human amniotic mesenchymal cell layer into (a) bone defect(s).

5. Use of a bone stem cell existing in human amniotic mesenchymal cell layer for osteogenesis.
